# EUROPEAN PATENT SPECIFICATION

(11) **EP 4 322 894 B1**
(45) Date of publication and mention of the grant of the patent: **03.06.2026**
(21) Application number: 22725530.4
(22) Date of filing: 11.04.2022
(51) Int. Cl.: A61F 5/44, A61F 5/00, A61F 5/445, A61M 16/04, A61M 25/10, A61M 25/04, A61M 25/00

(54) **CATHETER SYSTEM WITH PRESSURE MANAGEMENT DEVICE**
KATHETERSYSTEM MIT DRUCKVERWALTUNGSVORRICHTUNG
SYSTÈME DE CATHÉTER AVEC DISPOSITIF DE GESTION DE PRESSION

(30) Priority: 12.04.2021 US 202163173814 P; 10.05.2021 US 202163186528 P
(43) Date of publication of application: 21.02.2024
(73) Proprietor: ConvaTec Limited, Deeside, Flintshire CH5 2NU (GB)
(72) Inventor: TSAI, Mingliang Lawrence, Holmdel, NJ 07733 (US)
(74) Representative: Wilson Gunn
(86) International application number: PCT/IB2022/000204
(87) International publication number: WO 2022/219401

(56) References cited:
- EP-B1- 2 575 703
- WO-A1-2006/041496
- WO-A1-2016/087926
- US-A1- 2014 052 063
- US-A1- 2018 229 013
- US-B2- 9 808 606

## Description

### CROSS-REFERENCE TO RELATED DISCLOSURES

The present disclosure claims the benefit of U.S. Application No. 63/173,814 filed on April 12, 2021 and U.S. Application No. 63/186,528 filed on May 10, 2021.

### TECHNICAL FIELD

The present disclosure generally relates to catheters and associated systems, and more particularly but not exclusively relates to fecal catheters and associated systems.

### BACKGROUND

Indwelling fecal management catheters are often utilized to manage the liquid or semi-liquid fecal matter of non-ambulatory hospital patients. While indwelling fecal catheters are considered a standard in most intensive care units (ICUs) in managing non-ambulatory hospital patients who are fecal incontinent due to liquid or semi-liquid fecal matter, one patient risk in the use of an intrusive device, such as an indwelling fecal management catheter, is the increased rectal pressure exerted by the retention balloon. In order to anchor the retention balloon effectively, sufficient balloon inflation is necessary, giving rise to the increased level of balloon pressure, and thus rectal pressure. Such a high rectal pressure over an extended period of time during use of the device can lead to various adverse events, such as pressure ulcers and/or rectal bleeding. Therefore, these two factors are conflicting needs for the design of a retention balloon for essentially all indwelling catheters, including fecal, urinary, airway, and others.

US 2014/052063 A1 discloses a system as defined by the preamble of claim 1.

Various designs have been developed to address this unmet patient need. For example, European Patent No. 2,278,945 discloses the use of a fill indicator. More particularly, the '945 Patent discloses a rectal drainage appliance that includes a tubular element having an inflatable balloon at a distal end for anchoring the appliance in the rectum. The appliance can include first and second auxiliary lumens communicating with the inflatable balloon to provide independent inflation and pressure monitoring paths coupled to the balloon. The appliance can include a pressure state indicator defined by a mechanical element configured to flip between first and second states or shapes responsive to sensed pressure. The pressure state indicator may also be used in intestinal drains.

Another prior approach is disclosed in US Patent No. 8,939,952, which involves the use of double balloons including an air pocket. More particularly, the '952 Patent discloses a rectal appliance with a tubular member defining a communication passage for body waste, and first and second inflatable chamber portions carried on the tubular member for forming internal and external seals with respect to the anus. One or both of the inflatable chamber portions have a partly flared shape. The second inflatable chamber portion has a low external profile, and a concave sealing surface. The inflatable chamber portions are defined at least partly by a common flexible membrane that is constrained near a middle region to define a narrow waist between the two inflatable chamber portions.

Another prior approach involving the use of double balloons including an air pocket is disclosed in PCT Publication No. WO 2007/118621 A1. The '621 Publication discloses an occlusion system for management of rectal or anal incontinence, comprising a shaft element with a distal end and a proximal end and with a lumen extending all the way from the distal end to the proximal end of the shaft element, and a balloon which is mounted adhesively and sealingly on the shaft element and can be filled with a filling medium via a filling line. The balloon is formed from a tube section and is shaped in such a way that, when filled with the filling medium, it forms a first balloon configuration as intrarectal balloon and a second balloon configuration as abutment balloon, which are delimited from each other by a constriction. The distal end of the shaft element is designed with an end cap with a tip and, at a distance from the tip, at least one gas inlet opening is provided communicating with the lumen of the shaft element. The proximal end of the shaft element is connected to an outlet tube, which extends transverse to the longitudinal axis of the shaft element, has outlet openings at its ends, and communicates with the lumen.

Another prior approach is disclosed in European Patent No. 2,575,703, which generally discloses a catheter including a compressible foam. More particularly, the '703 Patent discloses a drainage appliance including a distal portion with a distensible retention cuff for retaining the distal portion in the rectum, intestinal or urinary tract. Cuff distension is controlled by a combination of: (a) a resiliently deformable device for urging distension of the cuff; (b) a fluid-containing chamber of variable size; and (c) a fluid-transfer damper for restricting fluid flow with respect to the chamber when the chamber changes size.

Many existing approaches, including those set forth in the above-referenced documents, may suffer from one or more drawbacks or limitations. For example, certain existing approaches can only reduce the risk of balloon over-inflation instead of the pressure that is present in standard use. Certain existing approaches fail to reduce the still-high balloon pressure, especially during bowel movement or patient movement when the retention balloon is compressed by the rectal wall. Certain existing approaches require the use of a fluid-transfer damper for restricting fluid flow into or out of the retention balloon, and the slow response of the fluid-transfer damper may not necessarily subside the relatively high balloon pressure quickly. For these reasons among others, there remains a need for further improvements in this technological field.

### SUMMARY

According to a first aspect of the present invention, there is provided a system in accordance with claim 1.

### BRIEF DESCRIPTION OF THE FIGURES

Fig. 1 is a cross-sectional illustration of a catheter according to certain embodiments.
Fig. 2 is a schematic illustration of a system according to certain embodiments, the system including the catheter illustrated in Fig. 1 and a pressure management device.
Fig. 3 is a cross-sectional illustration of the pressure management device illustrated in Fig. 2.
Fig. 4 is a schematic flow diagram of a process according to certain embodiments.

### DETAILED DESCRIPTION OF ILLUSTRATIVE EMBODIMENTS

Although the concepts of the present disclosure are susceptible to various modifications and alternative forms, specific embodiments have been shown by way of example in the drawings and will be described herein in detail.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. It should further be appreciated that although reference to a "preferred" component or feature may indicate the desirability of a particular component or feature with respect to an embodiment, the disclosure is not so limiting with respect to other embodiments, which may omit such a component or feature. Further, when a particular feature, structure, or characteristic is described in connection with an embodiment, it is submitted that it is within the knowledge of one skilled in the art to implement such feature, structure, or characteristic in connection with other embodiments whether or not explicitly described.

Additionally, it should be appreciated that items included in a list in the form of "at least one of A, B, and C" can mean (A); (B); (C); (A and B); (B and C); (A and C); or (A, B, and C). Similarly, items listed in the form of "at least one of A, B, or C" can mean (A); (B); (C); (A and B); (B and C); (A and C); or (A, B, and C). Items listed in the form of "A, B, and/or C" can also mean (A); (B); (C); (A and B); (B and C); (A and C); or (A, B, and C). Further, with respect to the claims, the use of words and phrases such as "a," "an," "at least one," and/or "at least one portion" should not be interpreted so as to be limiting to only one such element unless specifically stated to the contrary, and the use of phrases such as "at least a portion" and/or "a portion" should be interpreted as encompassing both embodiments including only a portion of such element and embodiments including the entirety of such element unless specifically stated to the contrary.

In the drawings, some structural or method features may be shown in certain specific arrangements and/or orderings. However, it should be appreciated that such specific arrangements and/or orderings may not necessarily be required. Rather, in some embodiments, such features may be arranged in a different manner and/or order than shown in the illustrative figures unless indicated to the contrary. Additionally, the inclusion of a structural or method feature in a particular figure is not meant to imply that such feature is required in all embodiments and, in some embodiments, may be omitted or may be combined with other features.

As used herein, the term "about" may be utilized to modify a quantitative representation that may permissibly vary from the stated value. In certain embodiments, the term "about" may signify a permissible variance of 10%. For example, a pressure described as "about 1.33 kPa (10 mmHg) above atmospheric pressure" may be in a range of 1.2 - 1.47 kPa (9-11 mmHg) above atmospheric pressure. Moreover, the term "atmospheric pressure" refers to standard atmospheric pressure (i.e., roughly 101.32 kPa (760 mmHg)) .

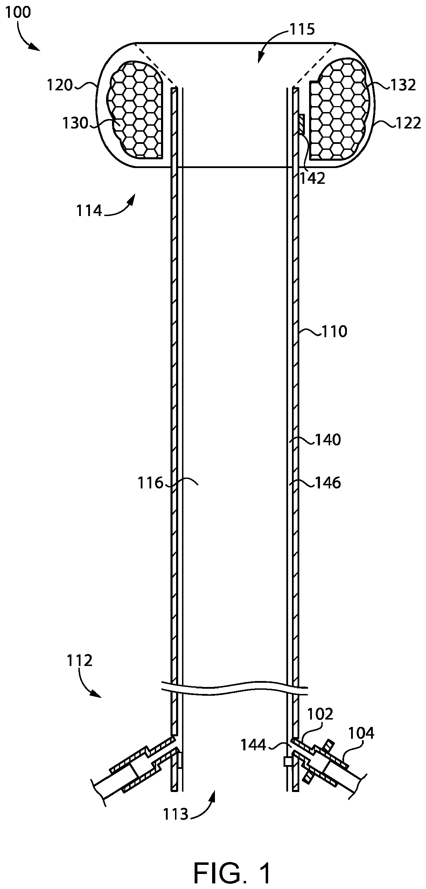

With reference to Fig. 1, illustrated therein is an indwelling catheter 100 according to certain embodiments. The catheter 100 generally includes a tubular body portion 110 having a proximal end portion 112 and an opposite distal end portion 114, a cuff 120 positioned at the distal end portion 114 of the body portion 110, a resilient device 130 positioned within a chamber 122 of the cuff 120, an inflation/deflation lumen 140 extending from the cuff 120 to an inflation/deflation port 102, and a first fitting 104 mounted to the inflation/deflation port 102. In the illustrated form, the indwelling catheter 100 is provided in the form of a fecal catheter, and the distal end portion 114 is configured for insertion into a patient's rectum. It is also contemplated that the catheter 100 may be provided in another form, such as that of a urinary catheter or an airway catheter.

The catheter body portion 110 extends from the proximal end portion 112 to the distal end portion 114, and defines an evacuation passage 116 having an inlet 115 formed in the distal end portion 114 and an evacuation port or outlet 113 formed in the proximal end portion 112. The passage 116 is configured to permit fluid (e.g., air, liquid, or semi-liquid) to flow from the inlet 115 to the outlet 113. For example, in the illustrated form, effluent will flow from the inlet 115 to the outlet 113 for discharge from the outlet 113. In certain embodiments, such as those in which the catheter 100 is utilized as an airway catheter, the passage 116 may further facilitate the flow of fluid (e.g., air) from the outlet 113 to the inlet 115, for example during inhalation by the patient.

The cuff 120 is positioned at the distal end portion 114 of the body portion 110, and defines a chamber 122 in which the resilient device 130 is housed. While other forms are contemplated, the illustrated cuff 120 is provided in the form of a substantially toroidal balloon that surrounds the inlet 115 of the evacuation passage 116. As described herein, the cuff 120 has a deflated state corresponding to a collapsed state of the resilient device 130 and an inflated state corresponding to an expanded state of the resilient device 130. During deflation of the cuff 120, the cuff 120 will compress or collapse the resilient device such that the resilient device stores mechanical energy that can subsequently be released to inflate the cuff 120.

The resilient device 130 is positioned in the chamber 122, and has a collapsed state and an expanded state. In the illustrated form, the resilient device 130 is provided in the form of an open-cell foam 132, such as an open-cell polyurethane foam. It is also contemplated that the resilient device 130 may be provided in another form, such as a form including one or more springs. As described herein, the resilient device 130 is configured to collapse during deflation of the cuff 120 for insertion of the cuff 120 into a cavity of the patient (e.g., the rectal cavity), and to thereafter expand to cause inflation of the cuff 120 for anchoring of the cuff 120 within the cavity.

The inflation/deflation lumen 140 has a distal end 142 that is open to the chamber 122, a proximal end 144 connected with the inflation/deflation port 102, and optionally an elongated portion 146 extending between and connecting the distal end 142 and the proximal end 144 such that the chamber 122 is in fluid communication with the inflation/deflation port 102. As a result, the cuff 120 is operable to be inflated and deflated via the first fitting 104 as described herein. The first fitting 104 may, for example, be provided in the form of a normally-closed check valve configured to transition to an open state when engaged with a mating syringe 220 or a pressure management device 210 as described herein.

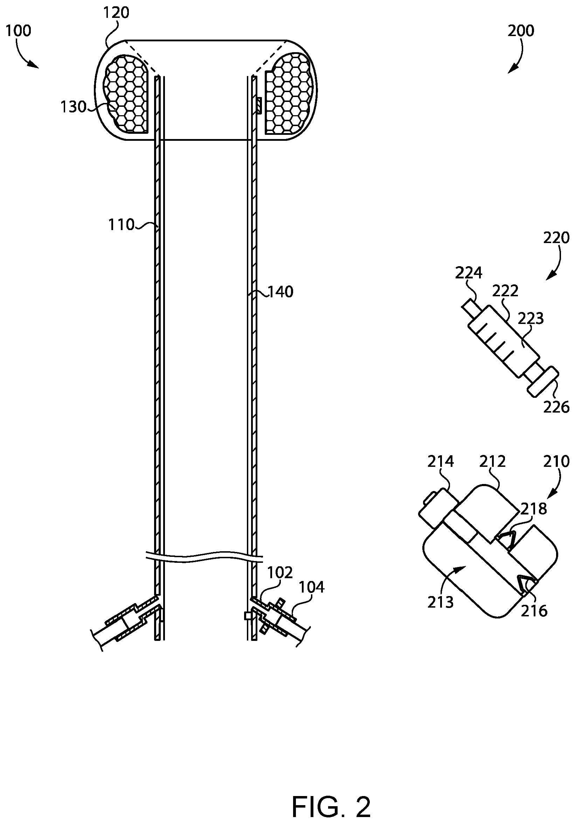

With additional reference to Fig. 2, illustrated therein is a system 200 according to certain embodiments. The system 200 includes the catheter 100 and a pressure management device 210 according to certain embodiments, and may further include a negative pressure generator such as a syringe 220. In certain embodiments, the pressure management device 210 may be tethered to the proximal end portion of the catheter 100 to discourage loss of the pressure management device 210. For example, the pressure management device 210 may include a strap by which the pressure management device 210 is tethered to a portion of the catheter 100 in the vicinity of the fitting 104 of the inflation/deflation port 102.

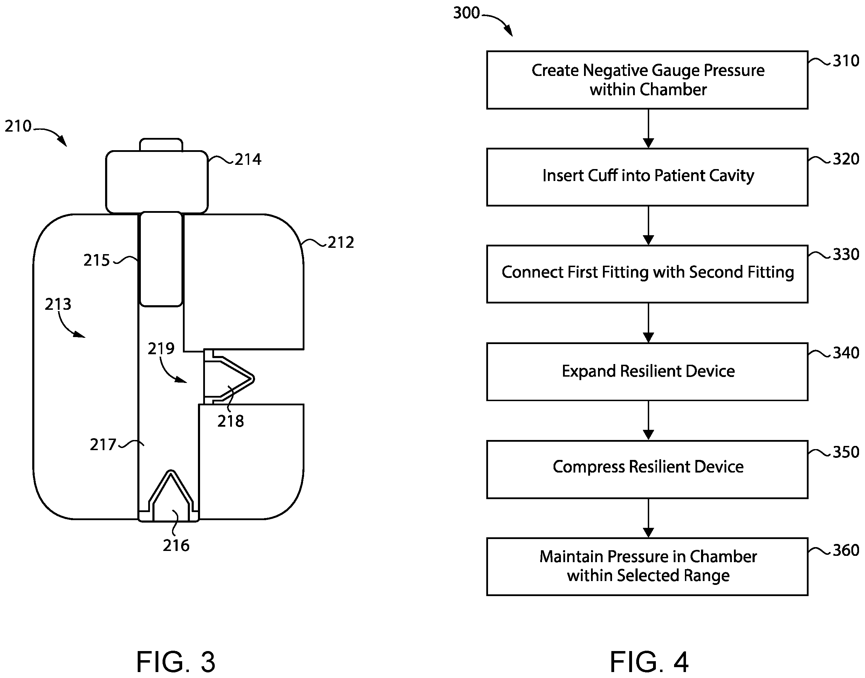

With additional reference to Fig. 3, the pressure management device 210 generally includes a body 212 defining a fluid passage 213, a second fitting 214 connected to a first branch 215 of the fluid passage 213, an inlet check valve 216 connected with a second branch 217 of the fluid passage 213, and an outlet check valve 218 connected with a third branch 219 of the fluid passage 213. In the illustrated form, the fluid passage 213 is provided in a generally T-shaped configuration, in which the third branch 219 is substantially perpendicular to the first branch 215 and the second branch 217. It is also contemplated that the fluid passage 213 may take another form. For example, the fluid passage 213 may be provided in a generally Y-shaped configuration in which the branches 215, 217, 219 extend at oblique angles relative to one another, or a V-shaped configuration in which the first branch 215 is omitted. The second fitting 214 is configured to mate with the first fitting 104 such that the fluid passage 213 is in communication with the inflation/deflation lumen 140 when the fittings 104, 214 are engaged with one another.

The inlet check valve 216 is normally closed, has a first cracking pressure, and is configured to permit fluid flow from a first pressure source to the fluid passage 213 when the pressure of the first pressure source exceeds the pressure within the fluid passage 213 by at least the first cracking pressure. When the pressure management device fitting 214 is secured to the catheter fitting 104, the pressure within the fluid passage 213 corresponds to the pressure within the cuff chamber 122. While other pressure sources are contemplated, in the illustrated embodiment, the first pressure source is ambient air. While the illustrated inlet check valve 216 is provided in the form of a duckbill valve, it is also contemplated that the inlet check valve 216 may be provided in another form, such as that of an umbrella valve, a disc valve, a diaphragm valve, or an open vent.

The outlet check valve 218 is normally closed, has a second cracking pressure, and is configured to permit fluid flow from the fluid passage 213 to a second pressure source when the pressure within the fluid passage 213 exceeds a pressure of the second pressure source by at least the second cracking pressure. As noted above, when the pressure management device fitting 214 is secured to the catheter fitting 104, the pressure within the fluid passage 213 corresponds to the pressure within the cuff chamber 122. While other pressure sources are contemplated, in the illustrated embodiment, the second pressure source is ambient air. While the illustrated outlet check valve 218 is provided in the form of a duckbill valve, it is also contemplated that the outlet check valve 218 may be provided in another form, such as that of an umbrella valve, a disc valve, or a diaphragm valve.

In certain embodiments, the inlet check valve 216 and the outlet check valve 218 may be selected with as the same type of check valve (e.g., one of a duckbill valve, an umbrella valve, a disc valve, a diaphragm valve, or an open vent). In certain embodiments, the inlet check valve 216 and the outlet check valve 218 may be selected as different types of check valves.

As described herein, the pressure management device 210 is configured to maintain pressure within the cuff chamber 122 within a predetermined pressure range having a minimum pressure corresponding to the cracking pressure of the inlet check valve 216 and a maximum pressure corresponding to the cracking pressure of the outlet check valve 218. In certain embodiments, the cracking pressure of the inlet check valve 216 may be between 0.67 kPa (5 mmHg) and 2.0 kPa (15 mmHg). In certain embodiments, the cracking pressure of the inlet check valve 216 may be about 1.3 kPa (10 mmHg) (e.g., 1.2 kPa (9 mmHg) to 1.5 kPa (11 mmHg)). In certain embodiments, the cracking pressure of the outlet check valve 218 may be about 4.0 kPa (30 mmHg) (e.g., 3.6 kPa (27 mmHg) to 4.4 kPa (33 mmHg)). In certain embodiments, the cracking pressure of the outlet check valve 218 may be about 2.7 kPa (20 mmHg) (e.g., 2.4 kPa (18 mmHg) to 2.9 kPa (22 mmHg)). In certain embodiments, the cracking pressure of the outlet check valve 218 may be about 1.3 kPa (10 mmHg) (e.g., 1.2 kPa (9 mmHg) to 1.5 kPa (11 mmHg)). In certain embodiments, the cracking pressure of the outlet check valve 218 may be 0.53-0.80 kPa (4-6 mmHg) or about 0.67 kPa (5 mmHg) (e.g., 0.60 kPa (4.5 mmHg) to 0.73 kPa (5.5 mmHg)).

The syringe 220 generally includes a body portion 212 that partially defines a fluid chamber 223, a third fitting 224, and a plunger 226 slidably mounted in the body portion 222. The third fitting 224 is configured to mate with the first fitting 104 such that the fluid chamber 223 is in communication with the inflation/deflation lumen 140 when the fittings 104, 224 are engaged with one another. When so engaged, retraction of the plunger 226 expands the fluid chamber 223, thereby creating a negative pressure in the cuff chamber 122. It is also contemplated that the negative pressure in the cuff chamber 122 may be generated in another manner, such as by use of a pump.

As noted above, each of the pressure management device fitting 214 and the syringe fitting 224 is configured to mate with the catheter fitting 104. Those skilled in the art will be readily capable of selecting fittings appropriate for use as the fittings 104, 214, 224. In certain embodiments, the catheter fitting 104 may be provided in the form of one of a female Luer fitting or a male Luer fitting, and each of the pressure management device fitting 214 and the syringe fitting 224 may be provided in the form of the other of a female Luer fitting or a male Luer fitting. For example, the catheter fitting 104 may be provided in the form of a female Luer fitting, and each of the pressure management device fitting 214 and the syringe fitting 224 may be provided in the form of a mating male Luer fitting. It should be appreciated that other forms of fittings are contemplated, and may be utilized to form the appropriate connections.

With additional reference to Fig. 4, an exemplary process 300 that may be performed using the system 200 is illustrated. Blocks illustrated for the processes in the present application are understood to be examples only, and blocks may be combined or divided, and added or removed, as well as re-ordered in whole or in part, unless explicitly stated to the contrary. Additionally, while the blocks are illustrated in a relatively serial fashion, it is to be understood that two or more of the blocks may be performed concurrently or in parallel with one another. Moreover, while the process 300 is described herein with specific reference to the system illustrated in Figs. 1 and 2, it is to be appreciated that the process 300 may be performed with systems having additional or alternative features.

The process 300 may begin with block 310, which generally involves creating a negative gauge pressure within a chamber having a resilient device positioned therein, thereby compressing the resilient device to a compressed state. In certain embodiments, the chamber is formed within a cuff, and an evacuation passage extends from the cuff to an evacuation port. In the illustrated form, block 310 involves creating a negative pressure in the chamber 122 of the cuff 120 using a negative pressure generation device, such as a syringe 220. More particularly, block 310 involves connecting the syringe fitting 224 to the catheter fitting 104, which is typically a one-way normally closed check valve, and retracting the plunger 226 to thereby create a negative gauge pressure within the chamber 122. It is also contemplated that block 310 may involve operating another form of negative pressure generation device to generate the negative pressure within the chamber 122. For example, block 310 may include operating a pump to generate the negative pressure within the chamber 122.

As the negative pressure is generated within the chamber 122, the cuff 120 moves from its inflated state to its deflated state, thereby causing the walls of the cuff 120 to compress the resilient device 130. As a result, the resilient device 130 transitions to its compressed state during deflation of the cuff 120. In its compressed state, the resilient device 130 stores mechanical energy that, when released, causes the resilient device 130 to expand to its expanded state, thereby expanding the cuff 120 as described herein.

In certain embodiments, the process 300 includes block 320, which generally involves inserting the cuff into a cavity of a patient. In the illustrated form, the catheter 100 is a fecal catheter, and block 320 involves inserting the cuff 120 into the rectum of the patient. It is also contemplated that block 320 may involve inserting the cuff 120 into another cavity of the patient, for example in the event that the catheter 100 is configured for use as a urinary catheter or an airway catheter.

The process 300 further includes block 330, which generally involves connecting a first fitting in fluid communication with the chamber with a second fitting of a pressure management device. In certain embodiments, the pressure management device comprises an inlet check valve in fluid communication with the second fitting, and an outlet check valve in fluid communication with the second fitting. In the illustrated form, block 330 involves connecting the inlet/outlet fitting 104 of the catheter 100 with the fitting 214 of the pressure management device 210, which includes an inlet check valve 216 and an outlet check valve 218. As noted above, each of the check valves 216, 218 is in fluid communication with the pressure management device fitting 214 via the fluid passage 213. In certain embodiments, such as those that include block 320, block 330 may be performed with the cuff 120 located in the patient cavity.

The process 300 further includes block 340, which generally involves expanding the resilient device from the compressed state to an expanded state, thereby distending the cuff and causing fluid to flow into the chamber via the inlet check valve. In the illustrated form, block 340 involves permitting the resilient foam 332 to expand from its compressed state to its expanded state, thereby distending the cuff 120. Cuff 120 remains deflated in block 310 and 320 despite the negative gauge pressure due to the nature of the one-way check valve in the first fitting 104. Upon connecting the pressure management device 210 with the first fitting 104 as described in block 330, negative pressure in the cuff 120 is then communicated to the pressure management device 210 via the inflation/deflation lumen 140. When the pressure differential across the inlet check valve 216 of the pressure management device 210 exceeds the cracking pressure of the inlet check valve 216, (which is normally closed) the inlet check valve 216 cracks, thereby permitting atmospheric air to flow into the chamber 122 for inflation of the cuff 120. Because the cuff 120 is still under a negative pressure during block 330 and block 340, the outlet check valve 218 remains closed. The inflow of fluid to the cuff 120 will continue until the cuff pressure reaches the cracking pressure of the inlet check valve 216, beyond which the inflow will automatically shut down. The use of a one-way inlet check valve 216 will permit the internal cuff pressure to remain at a negative pressure, or an atmospheric pressure at its limit, depending the choice of the cracking pressure selected.

The process 300 may further include block 350, which generally involves at least partially compressing the resilient device in response to an externally-applied force on the cuff, thereby at least partially collapsing the cuff as fluid flows out of the chamber via the outlet check valve. The externally-applied force may, for example, be the result of the patient's movement, such as due to a cough, sneeze, or bowel movement in which the rectum would contract in order to evacuate the fecal matter. In the illustrated form, block 350 involves at least partially compressing the resilient device 130 in response to such an externally-applied force on the cuff 120, thereby at least partially collapsing the cuff 120 as fluid flows out of the chamber 122 via the outlet check valve 218. Those skilled in the art will readily appreciate that when the external force is removed, the resilient device 130 once again expands to re-inflate the cuff 120 in a manner analogous to that described with reference to block 340. The outflow of the fluid in the cuff 120 decreases the cuff pressure. Because the cuff 120 is a positive pressure during block 350, the inlet check valve 216 remains closed. The outflow of fluid from the cuff 120 will continue until the cuff pressure reaches the cracking pressure of the outlet check valve 216, beyond which the outflow will automatically shut down. The use of a one-way outlet check valve will permit the internal cuff pressure to remain a positive pressure, or an atmospheric pressure at its limit, depending the choice of the cracking pressure selected.

The process 300 may further include block 360, which generally involves maintaining the pressure within the chamber in a selected pressure range having a minimum pressure and a maximum pressure. Block 360 may, for example, be performed by the pressure management device 210. In certain embodiments, the minimum pressure is between atmospheric pressure and about 2.0 kPa (15 mmHg) below atmospheric pressure. In certain embodiments, the minimum pressure is between 1.1 kPa (8 mmHg) below atmospheric pressure and 1.6 kPa (12 mmHg) below atmospheric pressure. In certain embodiments, the minimum pressure is between atmospheric pressure and about 1.3 kPa (10 mmHg) below atmospheric pressure.

In certain embodiments, the maximum pressure is about 4.0 kPa (30 mmHg) above atmospheric pressure or less. In certain embodiments, the maximum pressure is about 2.7 kPa (20 mmHg) above atmospheric pressure or less. In certain embodiments, the maximum pressure is about 1.3 kPa (10 mmHg) above atmospheric pressure or less. In certain embodiments, the maximum pressure is 0.53-0.80 kPa (4-6 mmHg) above atmospheric pressure. In certain embodiments, block 360 may involve maintaining the pressure within the chamber 122 in a range of 1.3 kPa (10 mmHg) below atmospheric pressure to 2.7 kPa (20 mmHg) above atmospheric pressure. In certain embodiments, block 360 may involve maintaining the pressure within the chamber 122 in a range of between 1.3 kPa (10 mmHg) below atmospheric pressure to 1.3 kPa (10 mmHg) above atmospheric pressure. In certain embodiments, block 360 may involve maintaining the pressure within the chamber 122 in a range of between 1.3 kPa (10 mm Hg) below atmospheric pressure to 0.67 kPa (5 mmHg) above atmospheric pressure. The ability for the cuff pressure to remain negative (i.e., below atmospheric pressure) while fully inflated one difference between existing approaches and certain embodiments of the disclosed subject matter. The external cuff pressure is a sum of the internal cuff pressure discussed above and the expansion force exerted by the resilient foam 130 onto the cuff 120, which may be about 1.3 kPa (10 mm Hg) or less based on the type of foam selected according to Table 1. Therefore, at least some embodiments of the present disclosure permit a maximum cuff pressure in contact of mucosal tissues of a body cavity with a maximum pressure of about 4.0 kPa (30 mmHg) above atmospheric pressure or less, or preferably, a maximum pressure of about 2.7 kPa (20 mmHg) above atmospheric pressure or less, or more preferably, a maximum pressure of about 1.3 kPa (10 mmHg) above atmospheric pressure or less.

Those skilled in the art will readily appreciate that the pressure range maintained within the chamber 122 depends at least in part upon the cracking pressure selected for the check valves 216, 218, and will readily be able to select check valves with appropriate cracking pressures to maintain a desired pressure range within the chamber 122. For example, in embodiments in which the minimum selected for the pressure within the chamber 122 is about 1.3 kPa (10 mmHg) below atmospheric, the inlet check valve 216 may be selected with a cracking pressure of about 1.3 kPa (10 mmHg) (e.g., 1.3 kPa (10 mmHg) +/-0.27 kPa (2 mmHg)). Similarly, in embodiments in which the maximum selected for the pressure within the chamber 122 is about 2.7 kPa (20 mmHg) above atmospheric, the outlet check valve 218 may be selected with a cracking pressure of about 2.7 kPa (20 mmHg) (e.g., 2.7 kPa (20 mmHg) +/- 0.53 kPa (4 mmHg)). Certain embodiments may utilize an open vent to allow for fast equilibrium to atmospheric pressure.

As should be appreciated from the foregoing, the system 200 including a catheter 100 and pressure management device 210 may be capable of accommodating patient movement while alleviating certain difficulties associated with prior approaches, such as patient discomfort and the risk of pressure ulcers. For example, when the patient moves, the increased pressure in the chamber 122 will act on the outlet check valve 218 to open and allow the pressure to subside quickly. The use of the one-way check valves 216, 218 may additionally or alternatively provide the benefit of quick pressure adjustment and conformity to the rectal wall, which may be correlated with improved sealing characteristics.

In certain embodiments, the system 200 may provide for better response times than prior approaches. For example, certain prior approaches required the use of a fluid-transfer damper that may be omitted in the currently-described system 200. Certain prior approaches that require the use of a fluid-transfer damper generally had response times in the range of ten to twenty minutes. In other words, such prior systems involving fluid-transfer dampers would take between ten and twenty minutes to allow for the cuff 120 and resilient device 130 to expand and/or contract. Such response times may be improved by certain embodiments of the system 200. For example, the system 200 may be configured such that the cuff 120 is able to move from its distended state to its collapsed state in two minutes or less, in one minute or less, or in thirty seconds or less. Such response times depend upon a number of factors, including the compressibility or resiliency of the resilient device 130 and/or the cracking pressure of the outlet check valve 218. Armed with the present disclosure, those skilled in the art will readily be capable of selecting these factors to provide the response times described above. Example parameters that may facilitate the reduction in response time and/or an increase in patient comfort are provided herewith as Table 1.

**Table 1: Example Parameters for Polyurethane Open Cell Foam**

| **Property** | **Unit** | **Testing Spec** | **Example Value** |
|---|---|---|---|
| Density | kg/m³ | DIN EN ISO 845 | 35 - 40 |
| Compression Load Deflection 40% | kPa | DIN EN ISO 3386-1 | 4-5 |
| Tensile Strength Dry | kPa | DIN EN ISO 1798 | 150 |
| Elongation Dry | % | DIN EN ISO 1798 | 170 |

The invention relates to a system in accordance with claim 1.

In certain embodiments, the pressure management device 210 lacks a fluid-transfer damper.

In certain embodiments, wherein the resilient device 130 comprises compressible foam 132.

In certain embodiments, each of the inlet check valve 216 and the outlet check valve 218 comprises at least one of a duckbill valve, an umbrella valve, a disc valve, or a diaphragm valve.

In certain embodiments, the outlet check valve 218 is configured to permit the cuff 120 to move from the inflated state to the deflated state in two minutes or less.

In certain embodiments, the outlet check valve 218 is configured to permit the cuff 120 to move from the inflated state to the deflated state in one minute or less.

In certain embodiments, the pressure management device 210 is configured to maintain a pressure within the chamber 122 between a minimum pressure and a maximum pressure; wherein the minimum pressure is between atmospheric pressure and 1.6kPa (12 mmHg) below atmospheric pressure; and wherein the maximum pressure is 4.0 kPa (30mmHg) above atmospheric pressure or less.

In certain embodiments, the maximum pressure is 2.7 kPa (20 mmHg) above atmospheric pressure or less.

In certain embodiments, the maximum pressure is 0.53-0.80 kPa (4-6 mmHg) above atmospheric pressure or less.

It should be understood that while the use of words such as preferable, preferably, preferred or more preferred utilized in the description above indicate that the feature so described may be more desirable, it nonetheless may not be necessary and embodiments lacking the same may be contemplated as within the scope of the invention, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

## Claims

1. A system, comprising:
a catheter (100), comprising:
a cuff (120) defining a chamber (122), the cuff (120) having a deflated state and an inflated state;
an evacuation passage (116) extending from the cuff (120) to an evacuation port (113); and
a first fitting (104) in fluid communication with the chamber (122); and
a pressure management device (210), comprising:
a second fitting (214) releasably engaged with the first fitting (104); and
at least one check valve (216, 218) in fluid communication with the second fitting (214),
wherein the at least one check valve (216, 218) comprises:
an inlet check valve (216) in fluid communication with the second fitting (214); and
an outlet check valve (218) in fluid communication with the second fitting (214),
wherein the inlet check valve (216) has a first cracking pressure and is configured to permit
fluid flow from a first pressure source to the chamber (122) when a pressure of the first
pressure source exceeds a pressure within the chamber (122) by at least the first cracking
pressure; and
wherein the outlet check valve (218) has a second cracking pressure and is configured to
permit fluid flow from the chamber (122) to a second pressure source when the pressure within
the chamber (122) exceeds a pressure of the second pressure source by at least the second
cracking pressure,
**characterized in that** a resilient device (130) is positioned in the chamber (122), the resilient device (130) having a compressed state corresponding to the deflated state and an expanded state corresponding to the inflated state, wherein the resilient device (130) is configured to expand from the compressed state to the expanded state to thereby cause the cuff (120) to move from the deflated state to the inflated state and **in that**
each of the first pressure source and the second pressure source is ambient air.

2. The system of claim 1, wherein each of the inlet check valve (216) and the outlet check valve (218) comprises at least one of a duckbill valve, an umbrella valve, a disc valve, or a diaphragm valve.

3. The system of claim 1, wherein the outlet check valve (218) is configured to permit the cuff (120) to move from the inflated state to the deflated state in two minutes or less.

4. The system of claim 3, wherein the outlet check valve (218) is configured to permit the cuff (120) to move from the inflated state to the deflated state in one minute or less.

5. The system of claim 1, wherein the resilient device (130) comprises compressible foam (132).

6. The system of claim 1, wherein the pressure management device (210) is configured to
maintain a pressure within the chamber (122) between a minimum pressure and a maximum pressure; wherein the minimum pressure is between atmospheric pressure and 1.6 kPa below
atmospheric pressure; and wherein the maximum pressure is 2.7 kPa above atmospheric pressure or less.

7. The system of claim 6, wherein the maximum pressure is 1.3 kPa above atmospheric pressure or less.

8. The system of claim 6, wherein the pressure within the chamber is a negative pressure between atmospheric pressure and 1.6 kPa below atmospheric pressure.

## Patentansprüche

1. System, umfassend:
einen Katheter (100), umfassend:
eine eine Kammer (122) definierende Manschette (120), wobei die Manschette (120) einen entleerten Zustand und einen aufgeblasenen Zustand aufweist;
einen sich von der Manschette (120) zu einer Evakuierungsöffnung (113) erstreckenden Evakuierungsdurchgang (116); und
ein mit der Kammer (122) in Fluidverbindung stehendes erstes Anschlussstück (104); und
eine Druckverwaltungsvorrichtung (210), umfassend:
ein mit dem ersten Anschlussstück (104) lösbar verbundenes zweites Anschlussstück (214); und
mindestens ein Rückschlagventil (216, 218) in Fluidverbindung mit dem zweiten Anschlussstück (214),
wobei das mindestens eine Rückschlagventil (216, 218) umfasst:
ein mit dem zweiten Anschlussstück (214) in Fluidverbindung stehendes Einlass-Rückschlagventil (216); und
ein mit dem zweiten Anschlussstück (214) in Fluidverbindung stehendes Auslass-Rückschlagventil (218),
wobei das Einlass-Rückschlagventil (216) einen ersten Öffnungsdruck aufweist und ausgebildet ist, um einen Fluidstrom von einer ersten Druckquelle zu der Kammer (122) zuzulassen, wenn ein Druck der ersten Druckquelle einen Druck innerhalb der Kammer (122) um mindestens den ersten Öffnungsdruck übersteigt; und
wobei das Auslass-Rückschlagventil (218) einen zweiten Öffnungsdruck aufweist und ausgebildet ist, um einen Fluidstrom von der Kammer (122) zu einer zweiten Druckquelle zuzulassen, wenn der Druck innerhalb der Kammer (122) einen Druck der zweiten Druckquelle um mindestens den zweiten Öffnungsdruck übersteigt, **dadurch gekennzeichnet, dass**
eine elastische Vorrichtung (130) in der Kammer (122) positioniert ist, wobei die elastische Vorrichtung (130) einen komprimierten Zustand entsprechend dem entleerten Zustand und einen expandierten Zustand entsprechend dem aufgeblasenen Zustand aufweist, wobei die elastische Vorrichtung (130) ausgebildet ist, um sich von dem komprimierten Zustand zu dem expandierten Zustand zu expandieren, um dabei zu bewirken, dass sich die Manschette (120) von dem entleerten Zustand in den aufgeblasenen Zustand bewegt, und dadurch, dass jede der ersten Druckquelle und der zweiten Druckquelle Umgebungsluft ist.

2. System nach Anspruch 1, wobei das Einlass-Rückschlagventil (216) und das Auslass-Rückschlagventil (218) mindestens eines von einem Duckbill-Ventil, einem Schirmventil, einem Scheibenventil oder einem Membranventil umfasst.

3. System nach Anspruch 1, wobei das Auslass-Rückschlagventil (218) ausgebildet ist, um zuzulassen, dass sich die Manschette (120) innerhalb von zwei Minuten oder weniger von dem aufgeblasenen Zustand in den entleerten Zustand bewegt.

4. System nach Anspruch 3, wobei das Auslass-Rückschlagventil (218) so ausgebildet ist, um zuzulassen, dass sich die Manschette (120) innerhalb von einer Minute oder weniger von dem aufgeblasenen Zustand in den entleerten Zustand bewegt.

5. System nach Anspruch 1, wobei die elastische Vorrichtung (130) komprimierbaren Schaum (132) umfasst.

6. System nach Anspruch 1, wobei die Druckverwaltungsvorrichtung (210) ausgebildet ist, um einen Druck innerhalb der Kammer (122) zwischen einem Minimaldruck und einem Maximaldruck zu halten;
wobei der Minimaldruck zwischen Atmosphärendruck und 1,6 kPa unterhalb des Atmosphärendrucks liegt; und
wobei der Maximaldruck 2,7 kPa über dem Atmosphärendruck oder weniger beträgt.

7. System nach Anspruch 6, wobei der Maximaldruck 1,3 kPa über dem Atmosphärendruck oder weniger beträgt.

8. System nach Anspruch 6, wobei der Druck innerhalb der Kammer ein Unterdruck zwischen Atmosphärendruck und 1,6 kPa unter Atmosphärendruck ist.

## Revendications

1. Un système, comprenant :
un cathéter (100), comprenant :
une manchette (120) définissant une chambre (122), la manchette (120) ayant un état dégonflé et un état gonflé ;
un passage (116) d'évacuation s'étendant de la manchette (120) à un orifice (113) d'évacuation ; et
un premier raccord (104) en communication fluidique avec la chambre (122) ; et
un dispositif (210) de gestion de la pression, comprenant :
un deuxième raccord (214) assemblé de manière amovible au premier raccord (104) ; et
au moins une soupape (216, 218) de non-retour en communication fluidique avec le deuxième raccord (214),
dans lequel la au moins une soupape (216, 218) de non-retour comprend :
une soupape (216) de non-retour d'entrée en communication fluidique avec le deuxième raccord (214) ; et
une soupape (218) de non-retour de sortie en communication fluidique avec le deuxième raccord (214),
dans lequel la soupape (216) de non-retour d'entrée a une première pression de claquage et est configurée pour permettre à un courant de fluide de passer d'une première source de pression à la chambre (122), lorsque la pression de la première source de pression dépasse une pression dans la chambre (122) d'au moins la première pression de claquage ; et
dans lequel la soupape (218) de non-retour de sortie a une deuxième pression de claquage et est configurée pour permettre un courant de fluide d'aller de la chambre (122) à une deuxième source de pression, lorsque la pression dans la chambre (122) dépasse une pression de la deuxième source de pression d'au moins la deuxième pression de claquage,
**caractérisé en ce qu'**
un dispositif (130) élastique est en position dans la chambre (122), le dispositif (130) élastique ayant un état comprimé correspondant à l'état dégonflé et un état détendu correspondant à l'état gonflé, dans lequel le dispositif (130) élastique est configuré pour passer de l'état comprimé à l'état détendu en faisant ainsi que la manchette (120) passe de l'état dégonflé à l'état gonflé et **en ce que** chacune de la première source de pression et de la deuxième source de pression est de l'air ambiant.

2. Le système de la revendication 1, dans lequel chacune de la soupape (216) de non-retour d'entrée et de la soupape (218) de non-retour de sortie comprend au moins une soupape à bec de canard, une soupape à parapluie, une soupape à disque ou une soupape à diaphragme.

3. Le système de la revendication 1, dans lequel la soupape (218) de non-retour de sortie est configurée pour permettre à la manchette (120) de passer de l'état gonflé à l'état dégonflé en deux minutes ou moins.

4. Le système de la revendication 3, dans lequel la soupape (218) de non-retour de sortie est configurée pour permettre à la manchette (120) de passer de l'état gonflé à l'état dégonflé en une minute ou moins.

5. Le système de la revendication 1, dans lequel le dispositif (130) élastique comprend de la mousse (132) compressible.

6. Le système de la revendication 1, dans lequel le dispositif (210) de gestion de la pression est configuré pour maintenir une pression dans la chambre (122) entre une pression minimum et une pression maximum ;
dans lequel la pression minimum est comprise entre la pression atmosphérique et 1,6 kPa en dessous de la pression atmosphérique ; et
dans lequel la pression maximum est de 2,7 kPa au-dessus de la pression atmosphérique ou moins.

7. Le système de la revendication 6, dans lequel la pression maximum est de 1,3 kPa au-dessus de la pression atmosphérique ou moins.

8. Le système de la revendication 6, dans lequel la pression dans la chambre est une dépression entre la pression atmosphérique et 1,6 kPa en dessous de la pression atmosphérique.
